Europäisches Patentamt

**European Patent Office**

Office européen des brevets

(11) Publication number:

# 0 090 463
# B1

# EUROPEAN PATENT SPECIFICATION

(45) Date of publication of patent specification: **22.06.88**

(51) Int. Cl.⁴: **A 61 F 5/00**

(21) Application number: **83200403.0**

(22) Date of filing: **24.03.83**

(54) **Accessory for counteracting the consequences of vesical incontinence with males.**

(30) Priority: **26.03.82 NL 8201258**

(43) Date of publication of application:
**05.10.83 Bulletin 83/40**

(45) Publication of the grant of the patent:
**22.06.88 Bulletin 88/25**

(84) Designated Contracting States:
**AT BE CH DE FR GB IT LI LU NL SE**

(56) References cited:
**DE-A-2 545 477**
**DE-A-2 545 577**
**DE-A-2 717 924**
**DE-C- 336 834**
**GB-A-1 019 286**
**GB-A-2 016 929**
**GB-A-2 036 561**

(73) Proprietor: **de Leur, Eric Jean Anne**
**Sydwende 9**
**NL-9204 KA Drachten (NL)**

(73) Proprietor: **Heijenga, Berend**
**Sluiskade NZ 194**
**NL-7603 NX Almelo (NL)**

(72) Inventor: **de Leur, Eric Jean Anne**
**Sydwende 9**
**NL-9204 KA Drachten (NL)**
Inventor: **Heijenga, Berend**
**Sluiskade NZ 194**
**NL-7603 NX Almelo (NL)**

(74) Representative: **Mommaerts, Johan Hendrik,
Dipl.-Phys.**
**Octrooibureau Lux Willem Witsenplein 4**
**NL-2596 BK Den Haag (NL)**

Courier Press, Leamington Spa, England.

## Description

The invention relates to an accessory for counteracting the consequences of vesical incontinence with males, as defined in the preamble of claim 1.

Such an accessory is known from DE—A 2 545 477. Therein the pressure element is a narrow lug with a more or less rounded surface, which lug is adapted to be pressed into the lower side of the penis so as to compress and thus, close the urethra. Although the penis is enclosed in a tubular casing which restricts the outer shape of the penis, it has appeared that the urethra may slip laterally away one side of the other, since the corpora cavernosa filling the greater part of the penis do not retain the urethra in its central position, and acts more or less as liquid masses without a definite outer shape. When the urethra slips away on the pressing surface, it is no longer sufficiently pinched for preventing urine from leaking away. It has appeared in practice that this known device does not work in an acceptable manner.

It is an object of the invention to provide such a device which does not show the latter draw-back, which device is characterised by the characterising part of claim 1.

Since, in the operative position, the inner and lower positions of the corpora cavernosa in the vicinity of the urethra are supported, the urethra can no longer slip away from its central position, and will be effectively pinched.

Further favourable embodiments of this accessory are defined in the appending subclaims.

It is remarked that, from DE 336 834 (1920) a device for the present purpose is known, which comprises a pressure element acting on the upper side of the penis, in which, however, in the operative position of said element, the penis is completely deformed. Apart from the fact that by acting on the upper side of the penis no effective pinching of the urethra can be obtained, this prior device is medically unacceptable since the penis will be excessively deformed, and the blood circulation in the penis will be seriously restricted or even completely interrupted thereby. Furthermore this device is intended to exert a pressure only in the middle part of the penis, and the lateral parts thereof are allowed to divert laterally without being supported, so that the urethra is not maintained in the central position, and, therefore, will not be effectively closed when sliding away from said central position.

From DE—A—2 717 924 another device is known, comprising a strap which can be tightened around the penis, and a small cushion made of foam material is provided on the inner side of said strap which, in the closed condition of said strap, is pressed against the lower side of the penis in order to pinch the urethra. However this foam cushions cannot provide a centered compression of the penis and neither the lateral support required for preventing a lateral movement of the urethra, and, moreover, in order to produce a compression force by means of such a cushion, the strap must be tightened to such an extent that blood vessels will be pinched too, and forming folds in the skin cannot be avoided either. Moreover the strap is fastened by means of an end portion which is to be pulled through a slot before being fixed by means of a barbed tape fastener, which impedes an easy and fast handling and adjustment.

Finally from GB—A 1 019 286 a device is known comprising a rigid strip having a central raised portion adapted to be pressed against the lower side of the penis, as well as a second flat rigid strip to be applied on the upper side of the penis, and the penis is compressed between said rigid strips by means of a flexible strap wrapped around said strips. The penis is not retained laterally, so that it can freely expand, and then the urethra will slip away from said raised portion and will no longer be compressed.

The invention will be elucidated below in more detail by reference to a drawing, showing in:

Fig. 1 a simplified diagrammatic cross-section of a penis;

Fig. 2 a section corresponding to Fig. 1 with an accessory not falling within the scope of the invention in the operative condition described for the purposes of explanation only:

Figs. 3A and B partial sections of a special embodiment of a part of the accessory according to the invention; and

Figs. 4A and B views, partly in section, of an embodiment of the accessory according to the invention in the inoperative and operative conditions resp.

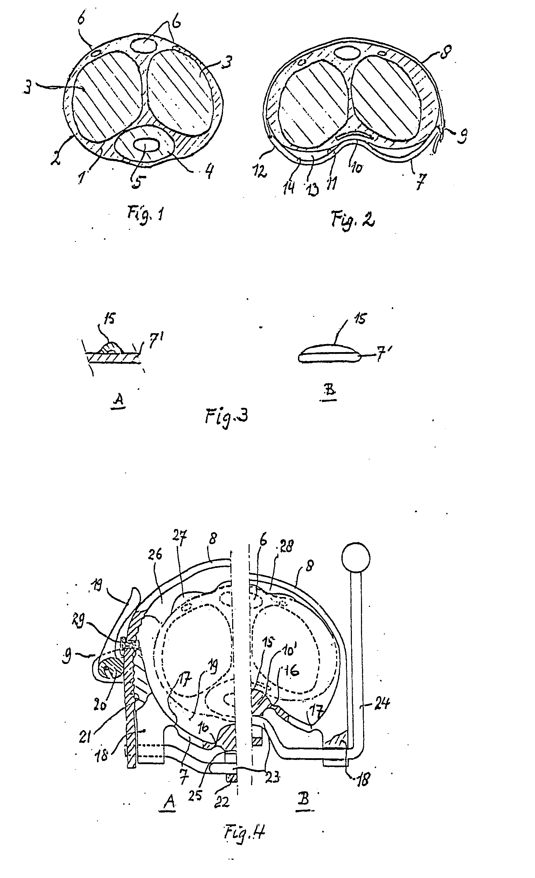

As diagrammatically shown in Fig. 1, a penis mainly consists of tissue 1 situated at the outer side with epidermis 2, within which two swelling bodies (corpora cavernosa) 3 and an intermediate spongy body (corpus spongiosum) 4 are present. In the latter body the urethra 5 is situated. Moreover blood vessels are present in the tissue 1, the major ones being shown at 6.

In Fig. 2 it is shown how the urethra 5 is compressed. To that end a pressure element 7 is used, consisting of a strip of relatively rigid plastics connected to a tensioning strap 8, the latter being adapted to be tightened by means of an easily releasable fastener 9. This fastener can be of any suitable type, e.g. as known in braces, luggage straps etc., and, if desired, also a barbed tape fastener can be used.

The strip 7 has a central bend 10 which, in the operative condition, presses the penis slight inwardly so that the spongy body 4 is pushed upwards. The urethra 5 is compressed and closed as shown, and is, then, stopped by the swelling bodies 3, and the latter can slightly be deforemed thereby. The whole will behave more or less as a liquid mass which, conserving its volume as determined by the tensioning strap 8, remains deformable, but the tissues can be slightly elastically compressed. The bodies 3 apply themselves somewhat against lateral parts 11 of the bend 10, and also the terminal parts 12 of the strip 7 can provide support.

The tensioning strap 8 provides for maintaining a sufficient counter-pressure. The fastener 9 is adjusted in such a manner that the urethra 5 is completely closed by compression, but the blood flow through the blood vessels 6 is not impeded.

In order to allow for some swelling of the penis, use can be made of a the flexibility of the strip 7, and the parts 12 can be made more flexible so that they will obtain, when being tightened, a corresponding curvature, and the interspaces 13 between the outer bends 14 of the strip 7 and/or the adjacent portion of the strap 8, on the one hand, and the epidermis 2, on the other hand, still allow for a local expansion of the penis, which, moreover, can be conducive to maintaining the blood flow in that portion.

The flexibility of the parts 12 allows for an adaptation to different penis dimensions.

Although the edges of the strip 7 are rounded, they can, nevertheless, be troublesome. Then it may be advisable to use, in the manner of Fig. 3, a strip 7' which is flatter in its central part, and to arrange, there, a pressing piece 15 which, as shown in Fig. 3B, is also curved in the axial plane, and, thus, provides a gradual transition towards the edges of the strip 7'. This pressing piece can, for example, be somewhat elastic, and, in particular, can be made hollow, and this piece can, for example, consist of a piece of rubber or plastic hose.

In Fig. 4 an embodiment of the accessory according to the invention is shown, which mainly comprises the same elements as the accessory of Fig. 2, which elements are indicated by the same reference numerals. The left-hand portion A and the right-hand portion B show the inoperative and operative conditions respectively.

The pressing strip 7 is subdivided by means of two pairs of flexible hinges 16 and 17, the central part 10' serving as the pressing body proper, which can be provided with an arched pressure piece 15, the outer hinges 17 each being connected to a thicker outer part 18, one of which being connected to the tensioning strap 8, which, in particular, can be extruded together therewith as one piece of plastics, the other outer part 18 being provided with the fastener 9. This fastener consists, in the case shown, of a lever 19 with an eccentric roll 20 forming a unitary structure therewith, and being adapted to press the strap 8, which may be provided with corrugations 21 in that region, against the adjacent surface of the outer part 18, which surface can be provided with similar corrugations so that, then, the strap can be unambiguously clamped.

The central part 10' is, at its lower side, provided with an open hook 22. In the gap between this hook 22 and the lower surface of the central part 10' a crank 23 is arranged, having extremities which are rotatable supported in the lateral parts 18, and one extremity is extended by an actuating lever 24 for actuating the crank 23.

In the operative position of Fig. 4B the bend of the crank 23 engages with a recess 25 in the lower side of the central part 10', so that the crank will be retained in this position. If necessary additional locking means can be provided for retaining the lever 24. The hook 22 serves to take along the central part 10' downwards when rotating the crank 23 towards the position of Fig. 4A.

In order to avoid that the blood vessels 6 will be pinched, it can be favourable to provide a pressing cushion 26 which is provided with rounded recesses 27 in the neighbourhood of these blood vessels, the intermediate portions 28 then transmitting the pressure towards the penis outside the region of the blood vessels 6.

The fastener 9 is adjusted in such a manner that, in the position of Fig. 4B, the urethra is effectively constricted, but the blood circulation is not impeded. This adjustment can, for instance, be experimentally determined, after which the strap 8 can be marked accordingly. This can, in particular, be done as shown by pinching in a rivet 29 preventing that the strap 8 will be pulled past the adjusted position. It will be clear that also other manners of marking are possible.

Such an accessory can be manufactured from plastic in a simple manner, and in particular the flexible strip 7 with the hinges 16 and 17 and the terminal parts 18, as well as the strap 8, can be extruded as one single piece. The crank 23 with the actuating lever 24 can be made, for instance, of metal wire, and can be snapped into suitable keyhole-shaped recesses of the lateral parts 18. The fastener 9 can be hooked or snapped on a pin formed on the lateral piece 18. In this manner the accessory can be quickly assembled, which will have a favourable influence on the retail price. Moreover such an accessory can be easily cleaned.

When using the embodiment of Fig. 2, the strap 8 is tightened to such an extent that the urethra is squeezed, and to bring about a urine discharge, the buckle or fastener 9 is to be loosened. Thereafter the strap 8 is tightened again. When using the embodiment of Fig. 4, the accessory is positioned on the penis with the crank in the position of Fig. 4A; thereafter the strap 8 is pulled through until the marking 29 has reached the fastener 9, and then the strap is fixed by means of said fastener. Subsequently the lever 24 can be shifted towards the position of Fig. 4B for squeezing the urethra. For bringing about a urine discharge, only the lever 24 has to be switched again, so that the central part 10' is moved downwards, and the urethra is opened thereby. At the end of the urine discharge, the lever 24 is returned towards the position of Fig. 4B. The fastener 9 is only to be actuated for removing the accessory from the penis.

Instead of the above-mentioned means for squeezing the urethra, also inflatable air cushions can be used by means of which the tensioning force can be adjusted at will by pumping in air, e.g. by means of a rubber ball, and by discharging the air by means of an adjustable valve. These air cushions can be provided under the central part 10 or 10′ or at the location of the pressing cushion 26. The operation of such an accessory does not differ from that of the embodiment described above. When using an air cushion, the releasable fastener 9 can sometimes be left out, and a local compression for avoiding the pinching of blood vessels can be obtained by a suitable design of the air cushion, or by subdividing said cushion into partial cushions.

The primary requirement is that the urethra will be sufficiently compressed for avoiding any urine discharge. The swelling bodies 3 are relatively hard, and the force provided by the strip 7, and in particular by the bend 10 thereof, should not be larger than required for compressing the spongy body 4, and, thus, the urethra 5. The shape of the bend 10 should, furthermore, be adapted to the shape of the swelling bodies 3 in such a manner that the urethra remains centered between these bodies. If the central part 10 is too steep, it may be possible that the urethra will slide away laterally, and then the compression thereof will become insufficient. The elastic compression of the spongy body is, in fact, sufficient for closing the urethra, so that too high pressures in other part of the penis, and in particular in the blood vessels, can be avoided. Additional foam cushions, e.g. as shown at 26 and/or laterally of the central part 10, can contribute, if required, to the desired pressure distribution and development.

Within the scope of the invention as defined by the claims, many modifications are possible.

## Claims

1. An accessory for counteracting the consequences of vesical incontinence with males, comprising an annular tensioning means (8) to be arranged around the penis of the user, and, which, in the closed condition, restricts the outer circumference of the penis, said means (8) comprising a pressure element (7) which is adapted to be moved transversely to the longitudinal axis of said tensioning means (8) between an outward inoperative position and an inner operative position, said pressure element (7) having a pressing surface (10) adapted to be placed against the lower part of the penis and to be pressed, in the operative position, into said part so as to constrict the urethra (4) of the penis, characterised in that the pressure element (7) consists of a strip which is subdivided by means of two sets of hinges (16, 17), the inner ones (16) delimiting a pressing body (10′) forming said pressing surface (10), and the outer ones (17) being connected to the tensioning means (8, 9), said pressing body (10′) engaging a crank (23) to be actuated by means of a lever (24), by means of which said body (10′) can be moved between the inoperative releasing and the operative, tightening positions, which crank (23) can be locked into the tightening position.

2. The accessory of claim 1, characterised in that the pressing body (10′) is provided with a retracting part (22) which grips around the crank (23).

3. The accessory of claim 1 or 2, in which the tensioning means is a strap (8) to be fixed by means of a fastener (9), and connected to the pressure element (7), characterised in that said strip (8) is provided, at the point corresponding to the highest tensioning force, with a stop means (29) which cannot be pulled past the fastener (9).

## Patentansprüche

1. Hilfsmittel zum Entgegenwirken der Folgen einer Blaseninkontinenz bei männlichen Personen, das ein ringförmiges Spannmittel (8), das um den Penis des Anwenders gelegt wird und das in geschlossenem Zustand den äusseren Umfang des Penis einschränkt, aufweist, wobei das Spannmittel (8) ein Druckelement (7) aufweist, das so eingerichtet ist, dass es transversal zur Längsachse des Spannmittels (8) zwischen einer äusseren Nicht-Arbeits-Stellung und einer inneren Arbeits-Stellung beweglich ist und wobei das Druckelement (7) eine Druckfläche (10) aufweist, die so eingerichtet ist, dass sie am unteren Teil des Penis anliegt und in Arbeitsstellung gegen diesen Teil des Penis drückt, so dass die Harnröhre (4) zusammengedrückt wird, dadurch gekennzeichnet, dass das Druckelement (7) aus einer Leiste besteht, die mittels zwei Sätzen Scharnieren (16, 17) unterteilt ist, wobei die inneren (16) einen Druckkörper (10′), welcher die Druckfläche (10) bildet, begrenzen und die äusseren (17) mit dem Spannmittel (8, 9) verbunden sind, wobei der Druckkörper (10′) in eine Kurbel (23) eingreift, die mittels eines Hebels (24) betätigt wird, vermittels dessen der Druckkörper (10′) zwischen der ausgeklinkten Nicht-Arbeits-Stellung und der angespannten Arbeits-Stellung bewegt werden kann und wobei die Kurbel (23) in der Arbeits-Stellung arretiert werden kann.

2. Hilfsmittel gemäss Patentanspruch 1, dadurch gekennzeichnet, dass der Druckkörper (10′) mit einem zurückziehenden Teil (22) versehen ist, der um den Hebel (23) herumgreift.

3. Hilfsmittel gemäss Patentanspruch 1 oder 2, worin das Spannmittel ein Spanngurt (8) ist, das mittels eine Verschlusses (9) befestigt wird und mit dem Druckelement (7) verbunden ist, dadurch gekennzeichnet, dass der Spanngurt (8) an der Stelle, die der höchstenn Zugkraft entspricht, mit einer Stopvorrichtung (29), welche nicht über den Verschluss (9) hinausgezogen werden kann, versehen ist.

## Revendications

1. Accessoire pour remédier aux conséquences de l'incontinence de la vessie masculine, comprenant des moyens annulaires de mise en tension (8) à placer autour du pénis de l'utilisateur et qui, dans l'état fermé, limitent la circonférence extérieure du pénis, lesdits moyens (8) comprenant un élément de pression (7) qui est prévu pour être déplacé transversalement à l'axe longitudinal desdits moyens de mise en tension (8) entre une position inactive vers l'extérieur et une position active intérieure, ledit élément de pression (7) comportant une surface de pression (10) prévue pour être placée contre la partie inférieure du pénis et pour être pressée, dans la position active, dans ladite partie de manière à pincer l'urètre (4) du pénis, caractérisé en ce que l'élément de pression (57) est constitué d'une bande qui est subdivisée par deux ensembles d'articulations (16, 17), les articulations intérieures (16) délimitant un corps de pression (10') qui présente ladite surface de pression (10), et les articulations extérieures (17) étant connectées aux moyens de mise en tension (8, 9), ledit corps de pression (10') étant en prise avec une manivelle (23) à actionner par un levier (24) au moyen duquel ledit corps (10') peut être déplacé entre la position de libération, inactive, et la position de serrage active, cette manivelle (23) pouvant être verrouillée dans la position de serrage.

2. Accessoire suivant la revendication 1, caractérisé en ce que le corps de pression (10') comporte une partie de rétraction (22) qui s'accroche autour de la manivelle (23).

3. Accessoire suivant la revendication 1 ou 2, dans lequel les moyens de mise en tension comprennent une lanière (8) à fixer par une attache (9) et connectée à l'élément de pression (7), caractérisé en ce que ladite lanière (8) est munie, au point correspondant à la force de mise en tension la plus élevée, d'un dispositif d'arrêt (29) qui ne peut pas être tiré au-delà de l'attache (9).

0 090 463

Fig. 1

Fig. 2

Fig. 3

Fig. 4